# EUROPEAN PATENT APPLICATION

(11) **EP 4 316 392 A2**
(43) Date of publication of application: **07.02.2024**
(21) Application number: 23216244.6
(22) Date of filing: 06.07.2020
(51) Int. Cl.: A61B 17/17

(54) **INSTRUMENTS AND METHODS FOR PREPARING A FEMUR IN A PARTIAL KNEE RECONSTRUCTION**

(30) Priority: 14.08.2019 US 201962886671 P
(62) Divisional of application: 20747279.6
(71) Applicant: Smith & Nephew, Inc., Memphis. Tennessee 38116 (US); Smith & Nephew Orthopaedics AG, 6300 Zug (CH); Smith & Nephew Asia Pacific Pte. Limited, Singapore 138565 (SG)
(72) Inventor: YEAGER, Jeffrey N., Tennessee, 38016 (US); CYKO, Christopher Ray, Tennessee, 38016 (US); JORDAN, Jason S., Tennessee, 38016 (US)
(74) Representative: Appleyard Lees IP LLP

(57) **Abstract**

A surgical instrument and accompanying methods for preparing a patient's femur to receive an orthopedic implant having a curved distal surface is disclosed. The femoral bone alignment device integrates a femoral alignment guide and a posterior cutting block with references to establish alignment to the distal femoral bone. That is, the bone alignment device includes a drill guide for establishing alignment of a unicompartmental femoral implant and a cutting slot for preparing the posterior bone. Corresponding methods optionally utilizing installation of an IM rod are also disclosed. During installation, the femoral bone alignment device may be used to pre-balance the patient's knee to establish alignment with respect to the femur concerning flexion-extension and varus-valgus alignment of the unicompartmental femoral implant. Meanwhile, anterior-posterior (A-P) placement and Superior-inferior (S-I) placement may also be established.

## Description

### CROSS-REFERENCE TO RELATED APPLICATION

This application claims the benefit of U.S. Provisional Patent Application No. 62/886,671, filed August 14, 2019, entitled "Instruments and Methods for Preparing a Femur in a Partial Knee Reconstruction," the entire contents of which application is hereby incorporated by reference in its entirety.

### FIELD OF THE DISCLOSURE

The present disclosure relates generally to orthopedic devices and instruments, and particularly to a device and/or instrument and accompanying methods for preparing a femur in a partial knee reconstruction.

### BACKGROUND OF THE DISCLOSURE

Partial knee replacement or arthroplasty (also known as unicompartmental knee arthroplasty or unicondylar knee arthroplasty) is a surgical procedure in which one of the medial or lateral compartment (sections) of a distal femur and a proximal tibia are replaced with an implanted orthopedic device. For example, partial knee replacement may be used to treat knee arthritis in patients who experience arthritis only in one compartment (section) of the knee joint, rather than throughout the knee joint. It can also be used to provide relief from pain and stiffness in patients who have medical conditions that aren't suitable for total knee replacement. In a partial knee replacement procedure, damaged cartilage and bone are removed and replaced only in the diseased compartment of a person's knee. This is in contrast to a total knee replacement, in which bone and cartilage from the entire joint are replaced.

Generally speaking, known orthopedic devices can be subdivided into resection and resurfacing devices. Resurfacing devices are typically balanced by establishing a desired laxity of the flexion space, then by alternating multiple trialing and reaming steps to establish a balanced knee, or desired amount of flexion and extension imbalance. Often, this requires multiple repetitive steps if enabling technology such as robotics or patient matched cutting blocks are not used. In addition, alignment of set-up blocks typically utilize a method that is disconnected from the patient's tibia, established by mechanical operation of the set-up device, or by disconnected devices. The devices used are typically offered in multiple sizes, representing characteristics specific to the size of the chosen implant, sizing is typically chosen by a different device in use previous to the balancing step, or after the extension space is prepared by a device.

As a result, known resurfacing devices and/or methods typically require a number of steps to prepare the femur including to determine proper flexion/extension space balancing, sizing, and bone preparation. For example, conventional methods may use, prior to bone preparation, sizing guides or spoons to establish component size, which may then be used to determine the proper alignment guide. Alternatively, sizing and alignment guides may be established by drill guides after the bone has been prepared.

Alignment of the component or orthopedic implant (flexion/extension and valgus alignment) may be established by mechanical reference to an intramedullary alignment rod. Alternatively, in methods without any mechanical coupling, with the use of a distinct and separate alignment guide. In addition, some methods establish flexion/extension and valgus alignment by eye. Rotational alignment may be established either independently (e.g., by eye), by mechanical operation of a guide block with reference to the patient's tibia, or by distension of the joint by a non-interconnected spacer. It is also known to utilize a mechanical alignment device connected to a set up block resting on the tibia, or with additional shims positioned between the alignment device and the patient's tibia.

Establishment of joint balance may be determined by provisionally preparing (e.g., reaming) then alternately trialing and additional reaming as necessary. Alternatively, it is known to utilize a saw and alternating with trialing, to utilize spacers interconnected with a set up block, which rests on the patient's tibia.

The bone may be prepared using sequential spherical reaming, sawing, progressive range of motion milling, range of motion reaming, etc. The bone/flexion space may be prepared by utilizing a separate set-up block, with orientation provided by the set-up device, a saw combined with extension space preparation, progressive range of motion milling combined with extension space preparation, a separate set-up block with orientation provided by extension space preparation, a separate resector prior to extension space preparation whereby the un-resected tibia is referenced with respect to joint tension and flexion/extension orientation is set by eye.

One disadvantage with known partial knee devices and methods is the relatively larger number of surgical steps and instruments needed to prepare the patient's bone to receive the orthopedic implant.

Thus, it would be beneficial to provide improved, simplified instruments and methods for preparing a patient's bone to receive an orthopedic implant in a partial knee reconstruction.

### SUMMARY OF THE DISCLOSURE

This Summary is provided to introduce a selection of concepts in a simplified form that are further described below in the Detailed Description. This Summary is not intended to identify key features or essential features of the claimed subject matter, nor is it intended as an aid in determining the scope of the claimed subject matter.

The present disclosure provides an improved surgical instrument and accompanying methods to prepare a femur to receive an implant with fewer surgical steps. The instrument and/or methods may facilitate and/or incorporate pre-balancing of the patient's knee (e.g., measuring space between the patient' unprepared femoral bone and the patient's prepared tibial bone including, for example, in flexion/extension direction) such that flexion and extension space balance, bone preparation, and sizing can be completed with a combined, integrated femoral bone alignment device or instrument. In one embodiment, the integrated bone alignment/sizing device is used to prepare the distal and posterior portions of a patient's femoral bone. In use, the integrated bone alignment/sizing device can be used to determine a location, placement, alignment, etc. (terms used interchangeably herein) of the femoral component (e.g., unicompartmental femoral implant) relative to joint tension with reference to a prepared tibia surface. Sizing of the femoral component can be determined/confirmed by provided references on the integrated bone alignment/sizing device. Posterior resection/joint space establishment can also be completed using the integrated bone alignment/sizing device. In addition, femoral alignment can be set with respect to anatomic landmarks, with or without reference to an intramedullary alignment rod.

In one embodiment, the integrated femoral bone alignment device includes a femoral alignment guide (e.g., a drill guide) and a cutting or sawing slot. In addition, the integrated femoral bone alignment device may include one or more references to establish alignment relative to the unprepared distal and posterior femoral bone.

In one embodiment, the integrated femoral bone alignment device includes one or more alignment or drill guides for establishing alignment of a unicompartmental femoral implant. In addition, the integrated femoral bone alignment device may include one or more cutting slots for preparing a portion of the patient's femoral bone such as, for example, the posterior portion of the patient's femoral bone.

In one embodiment, the integrated femoral bone alignment device also includes a tongue, projection or foot extending from a rear surface of the device for positioning in the space between the patient's unprepared femoral bone and the patient's tibial bone for provisionally positioning the integrated femoral bone alignment device against the patient's bones.

In one embodiment, the integrated femoral bone alignment device includes one or more holes or openings for connecting the integrated femoral bone alignment device to an optional intramedullary alignment rod.

In one embodiment, an alignment guide may be used to couple the integrated femoral bone alignment device to the intramedullary alignment rod.

In one embodiment, the integrated femoral bone alignment device may also include one or more anatomical reference markers to assist the surgeon in determining one or more degrees of freedom. For example, the integrated femoral bone alignment device may include a first anatomical reference marker extending along a proximal or top end of the device. In addition, the integrated femoral bone alignment device may include a second anatomical reference marker extending along a front surface of the device.

In one embodiment, the integrated femoral bone alignment device may also include a coupling mechanism for coupling, engaging, etc. one or more shims or spacers for positioning the integrated femoral bone alignment device into the space between the patient's prepared tibial bone and unprepared femoral bone to help establish alignment. In one embodiment, the coupling mechanism may be in the form of a T-shaped member for slidably receiving the shim or spacer.

In one embodiment, the integrated femoral bone alignment device may also include an indicator having a tip for contacting the patient's bone. In use, the indicator ensures that the device does not sit proud (or too deep) of the anatomy after bone preparation is completed.

In one embodiment, an integrated femoral bone alignment device arranged and configured to prepare a patient's unprepared femoral bone in a partial knee reconstruction surgery to receive a unicompartmental femoral implant is disclosed. The device comprises a top end, a bottom end, a front surface, a rear surface, a medial side surface, and a lateral side surface; a femoral alignment drill guide extending from the rear surface to the front surface, the femoral alignment drill guide arranged and configured to form an alignment hole in a distal end of the patient's femoral bone to establish alignment of the unicompartmental femoral implant; a cutting slot extending from the rear surface to the front surface, the cutting slot being arranged and configured to receive a surgical cutting instrument for resecting a portion of the patient's unprepared femoral bone; and one or more anatomical reference markers to establish alignment of the integrated femoral bone alignment device, and hence the unicompartmental femoral implant, relative to patient's unprepared femoral bone.

In one embodiment, the one or more anatomical reference markers include a first anatomical reference marker extending along a top end of the device from the rear surface towards the front surface; and a second anatomical reference marker extending along a front surface of the device from the top end to the bottom end. In one embodiment, during use, the first and second anatomical reference markers assist a surgeon determine internal-external (I-E) and medial-lateral (M-L) placement of the unicompartmental femoral implant.

In one embodiment, the anatomical reference markers consists one of a series of lines, grooves, slots, markings, or a combination thereof.

In one embodiment, the integrated femoral bone alignment device further comprises a tongue extending from the rear surface, the tongue being arranged and configured to be positioned in a space between the patient's unprepared femoral bone and the patient's prepared tibial bone for provisionally positioning the integrated femoral bone alignment device against the patient's femoral bone. In one embodiment, a distance between the tongue and the cutting slot is a fixed distance, such that resecting a portion of the patient's unprepared femoral bone removes a known thickness of the patient's femoral bone. In one embodiment, positioning the tongue in the space between the patient's unprepared femoral bone and the patient's tibial bone establishes anterior-posterior (A-P) placement of the unicompartmental femoral implant.

In one embodiment, the integrated femoral bone alignment device further comprises one or more openings formed in the front surface thereof; and an alignment guide arranged and configured to be positioned within the one or more openings for coupling the integrated femoral bone alignment device to an alignment rod referencing the patient's femoral bone. In one embodiment, the integrated femoral bone alignment device includes first and second openings positioned on either side of the integrated femoral bone alignment device. In one embodiment, in use, coupling the alignment guide with the integrated femoral bone alignment device and the alignment rod establishes flexion-extension (F-E) and varus-valgus (V/V) alignment of the unicompartmental femoral implant.

In one embodiment, the integrated femoral bone alignment device further comprises a coupling mechanism for one or more shims, the one or more shims being arranged and configured to slidably engage with the coupling mechanism for positioning the integrated femoral bone alignment device into a space between the patient's prepared tibial bone and the patient's unprepared femoral bone establishes alignment of the unicompartmental femoral implant. In one embodiment, the coupling mechanism is in the form of a T-shaped member for slidably receiving the one or more shims. In one embodiment, the coupling mechanism is in the form of a slot or female connection for slidably receiving the one or more shims.

In one embodiment, the integrated femoral bone alignment device further comprises an indicator having a tip for contacting the patient's bone.

In one embodiment, the integrated femoral bone alignment device further comprises one or more additional drill guides arranged and configured to receive a surgical drill to prepare the patient's bone to receive a fixation feature for the unicompartmental femoral implant.

In one embodiment, the integrated femoral bone alignment device further comprises one or more holes arranged and configured to receive a fixation member for provisionally coupling the integrated femoral bone alignment device to the patient's unprepared femoral bone. In one embodiment, the fixation member is a pin for provisionally coupling the integrated femoral bone alignment device to the patient's unprepared femoral bone.

In one embodiment, the integrated femoral bone alignment device comprises an outer profile that is asymmetric and arranged and configured for use with either a medial or a lateral portion of the patient's femoral bone.

In one embodiment, superior-inferior (S-I) placement of the unicompartmental femoral implant is established by contacting the rear surface of the integrated femoral bone alignment device with the patient's unprepared femoral bone.

Exemplary methods of preparing a patient's femur are also disclosed herein. In one method, an integrated femoral bone alignment device incorporating an alignment guide (e.g., drill guide) and a cutting slot (e.g., a posterior cutting slot) is used. In one example method for preparing a distal portion of a patient's femoral bone, an access hole is initially drilled into a distal end of the patient's femur. Thereafter, an intramedullary alignment rod is positioned into the access hole. Next, an integrated femoral bone alignment device is provisionally positioned against the patient's unprepared femoral bone. An alignment device is subsequently positioned and coupled to the integrated femoral bone alignment device and to the intramedullary alignment rod to establish alignment with respect to the femur.

Subsequently, using the drill guide formed in the integrated femoral bone alignment device, a drill bit may be used to create an alignment hole in the distal portion of the patient's femur. Using the cutting slot formed in the integrated femoral bone alignment device, a saw or cutting blade may be used to resect portions of the patient's bone such as, for example, the posterior portion of the patient's femur.

Finally, the integrated femoral bone alignment device may be removed and the distal portion of the patient's femur may be prepared using the established reference points including the alignment hole to position an alignment shaft for receiving a rotary mill or cutting tool. Femoral trialing may be completed, and an orthopedic implant may be implanted.

In one embodiment, the integrated femoral bone alignment device is provisionally positioned against the patient's unprepared femoral bone by inserting a tongue extending from a rear surface of the integrated femoral bone alignment device into the space between the patient's unprepared femur and the patient's tibia.

In one embodiment, coupling the alignment device with the integrated femoral bone alignment device and the intramedullary alignment rod establishes flexion-extension (F-E) and varus-valgus (V/V) alignment of the unicompartmental femoral implant.

In one embodiment, anterior-posterior (A-P) placement may be established by contact with the tongue extending from the rear surface of the integrated femoral bone alignment device.

In one embodiment, superior-inferior (S-I) placement is established by contact with the rear surface of the integrated femoral bone alignment device.

In one embodiment, the remaining degrees of freedom including internal-external (I-E) and medial-lateral (M-L) placement may be established by hand utilizing one or more anatomical reference markers on the integrated femoral bone alignment device.

In one embodiment, creating the alignment hole in the distal portion of the patient's femur may be used by a preparation device to establish extension space balance.

In one embodiment, resecting portions of the posterior portion of the patient's femur establish flexion space balance.

In another example method for preparing a distal portion of a patient's femoral bone, the integrated femoral bone alignment device is initially provisionally positioned against the patient's unprepared femoral bone. One or more shims or spacers may be positioned between the integrated femoral bone alignment device and the patient's prepared tibial bone to assist with establishing alignment with respect to the patient's femur.

Next, using the drill guide formed in the integrated femoral bone alignment device, a drill bit is used to create an alignment hole in the distal portion of the patient's femur. Using the cutting slot formed in the integrated femoral bone alignment device, a saw or cutting blade may be used to resect portions of the posterior portion of the patient's femur.

Finally, the integrated femoral bone alignment device may be removed and the distal portion of the patient's femur may be prepared using the established reference points including the alignment hole to position an alignment shaft for receiving a rotary mill or cutting tool. Femoral trialing may be completed, and an orthopedic implant may be implanted.

In one embodiment, the integrated femoral bone alignment device is initially provisionally positioned by inserting a tongue extending from a rear surface of the device into the space between the patient's unprepared femur and the patient's tibia.

In one embodiment, the shim may be coupled to the integrated femoral bone alignment device via a coupling mechanism formed on the integrated femoral bone alignment device. Thereafter, the shim may be positioned within the space between the patient's prepared tibial bone and the patient's unprepared femoral bone.

In one embodiment, a method for preparing a patient's femoral bone for use in a partial knee reconstruction surgery to implant a unicompartmental femoral implant is disclosed, in which the patient's knee has an existing imbalance in the patient's unprepared femoral bone in extension as compared to flexion. The method comprises positioning a tongue extending from a rear surface of an integrated femoral bone alignment device into a patient's knee space between the patient's unprepared femoral bone and the patient's prepared tibial bone, the integrated femoral bone alignment device further including an alignment guide and a cutting slot; inserting a drill into the alignment guide to form an alignment hole in a distal end of the patient's unprepared femoral bone; inserting a saw blade into the cutting slot to resect a posterior portion of the patient's femoral bone; removing the integrated femoral bone alignment device; and preparing the patient's femur utilizing the alignment hole.

In one embodiment, the method further comprises forming an access hole into a distal end of the patient's unprepared femoral bone; inserting an alignment rod into the access hole; and coupling an alignment device to the integrated femoral bone alignment device and to the alignment rod. In one embodiment, coupling the alignment device to the integrated femoral bone alignment device and to the alignment rod comprises inserting a projection formed on a first end of the alignment device into a hole formed in the integrated femoral bone alignment device; and positioning a fork-like second end portion of the alignment device about the alignment rod. In one embodiment, coupling the alignment device to the integrated femoral bone alignment device and the alignment rod establishes flexion-extension (F-E) and varus-valgus (V/V) alignment of the unicompartmental femoral implant.

In one embodiment, the method further comprises inserting an alignment shaft into the alignment hole; and positioning a rotary cutting tool over the alignment shaft to prepare the patient's unprepared femoral bone.

In one embodiment, the method further comprises inserting one or more shims within the patient's knee space between the integrated femoral bone alignment device and the patient's prepared tibial bone to establish alignment of the unicompartmental femoral implant with respect to the patient's femoral bone, the one or more shims being coupled to the integrated femoral bone alignment device.

In one embodiment, a distance between the tongue and the cutting slot is a fixed distance such that resecting a posterior portion of the patient's unprepared femoral bone removes a known thickness of the patient's femoral bone.

In one embodiment, the integrated femoral bone alignment device further includes a first anatomical reference marker extending along a top end of the integrated femoral bone alignment device and a second anatomical reference marker extending along a front surface of the integrated femoral bone alignment device, the method further comprises using the first and second anatomical reference markers to establish internal-external (I-E) and medial-lateral (M-L) placement of the unicompartmental femoral implant.

In one embodiment, positioning the tongue into the patient's knee space establishes anterior-posterior (A-P) placement of the unicompartmental femoral implant.

In one embodiment, positioning the tongue into the patient's knee space comprises contacting a rear surface of the integrated femoral bone alignment device with the patient's unprepared femoral bone to establish superior-inferior (S-I) placement of the unicompartmental femoral implant.

By utilizing an integrated femoral bone alignment device alignment of the femoral component (e.g., unicompartmental femoral implant) can be accomplished in an easier surgical procedure requiring fewer surgical steps. In addition, pre-balancing the patient's knee can be accomplished so that flexion and extension space balance, bone preparation, and sizing can be completed with a single integrated device.

Further features and advantages of at least some of the embodiments of the present invention, as well as the structure and operation of various embodiments of the present invention, are described in detail below with reference to the accompanying drawings.

### BRIEF DESCRIPTION OF THE DRAWINGS

By way of example, a specific embodiment of the disclosed device will now be described, with reference to the accompanying drawings, in which:
**FIG. 1** illustrates a perspective view of an example of an embodiment of a femoral bone alignment device in accordance with the principles of the present disclosure;
**FIG. 2** illustrates various perspective views representing a patient's knee undergoing partial knee reconstruction, the patient's knee incorporating an imbalance between the patient's unprepared femoral bone and the patient's prepared tibial bone in the flexion/extension direction;
**FIGS. 3A-3G** illustrate various perspective views of various steps used in connection with an example method of preparing a patient's femoral bone in a partial knee reconstruction procedure;
**FIGS. 4A-4E** illustrate various perspective views of various steps used in connection with an alternate example method of preparing a patient's femoral bone in a partial knee reconstruction procedure; and
**FIGS. 5A-5D** illustrate various views of an alternate example of an embodiment of a femoral bone alignment device in accordance with the principles of the present disclosure.

The drawings are not necessarily to scale. The drawings are merely representations, not intended to portray specific parameters of the disclosure. The drawings are intended to depict various embodiments of the disclosure, and therefore are not to be considered as limiting in scope. In the drawings, like numbering represents like elements.

### DETAILED DESCRIPTION

Embodiments of an improved integrated femoral bone alignment device for use in preparing a patient's femoral bone in a partial knee reconstruction will now be described more fully hereinafter with reference to the accompanying drawings, in which preferred embodiments of the present disclosure are presented. As will be described and illustrated, in some embodiments, the integrated femoral bone alignment device includes an alignment guide (e.g., drill guide) and a cutting or sawing slot. In addition, the integrated femoral bone alignment device may include references to establish alignment to the unprepared distal and posterior femoral bone. The integrated femoral bone alignment device of the present disclosure may, however, be embodied in many different forms and should not be construed as being limited to the embodiments set forth herein. Rather, these embodiments are provided so that this disclosure will convey certain example embodiments of the device and method to those skilled in the art. In the drawings, like numbers refer to like elements throughout unless otherwise noted.

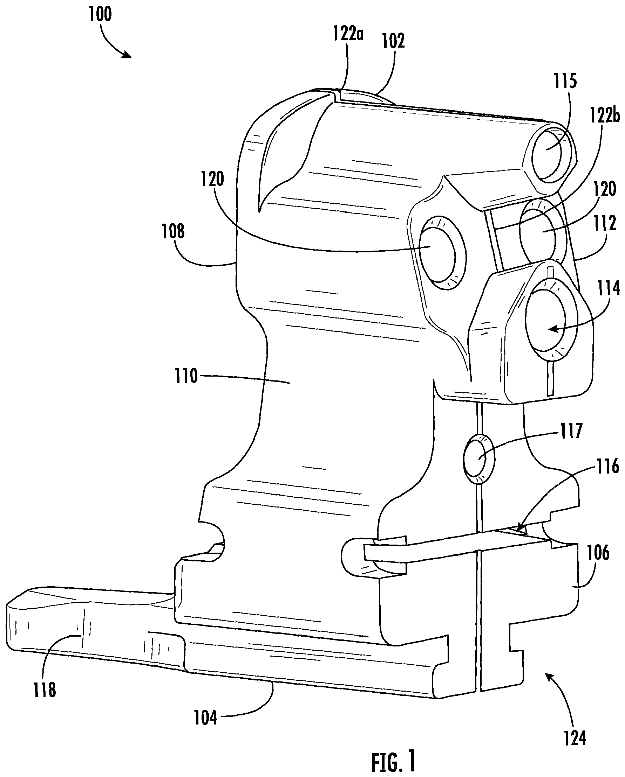

**FIG. 1** illustrates an embodiment of a bone alignment device such as, for example, an integrated femoral bone alignment device 100 for use in preparing a patient's femur or femoral bone undergoing a partial knee reconstruction procedure. As shown, and as will be described in greater detail, the integrated femoral bone alignment device 100 integrates a femoral alignment guide (e.g., a drill guide), a cutting or sawing slot, and references to establish alignment to the unprepared distal and posterior femoral bone.

As shown, the integrated femoral bone alignment device 100 includes an anterior or top end 102, a posterior or bottom end 104, a front surface 106, a rear surface 108, a medial or first side surface 110, and a lateral or second side surface 112. In addition, as shown, the integrated femoral bone alignment device 100 may also include one or more alignment or drill guides 114 for establishing alignment of a unicompartmental femoral implant, as will be described in greater detail below. As shown, the one or more drill guides 114 extend from the rear surface 108 to the front surface 106.

In addition, and/or alternatively, as shown, the integrated femoral bone alignment device 100 may also include one or more cutting slots 116 for preparing a portion of the patient's femoral bone, as will be described in greater detail below. As shown, the cutting slot 116 may extend from the rear surface 108 to the front surface 106, and from the first side surface 110 to the second side surface 112.

In addition, and/or alternatively, as shown, the integrated femoral bone alignment device 100 may also include a tongue, projection or foot 118 (used interchangeably herein without the intent to limit) extending from the rear surface 108 of the device 100. As will be described in greater detail below, during use, the tongue 118 is arranged and configured to be positioned in the space between the patient's unprepared femoral bone and the patient's tibial bone for provisionally positioning the integrated femoral bone alignment device 100 against the patient's bones.

In addition, and/or alternatively, the integrated femoral bone alignment device 100 may include one or more holes or openings 120 for connecting the integrated femoral bone alignment device 100 to an optional intramedullary alignment rod, as will be described in greater detail below. As shown, the one or more holes or openings 120 may extend from the front surface 106 towards the rear surface 108. As shown, the integrated femoral bone alignment device 100 may include first and second holes or openings 120 positioned on either side of the integrated femoral bone alignment device 100. In use, the appropriate hole or opening 120 may be selected for coupling to the optional intramedullary alignment rod depending on whether the medial or lateral compartment of the patient's bone is being prepared.

In addition, and/or alternatively, as shown, the integrated femoral bone alignment device 100 may also include one or more anatomical reference markers 122 to assist the surgeon in determining one or more degrees of freedom. For example, as shown, the integrated femoral bone alignment device 100 may include an anatomical reference marker 122a extending from the rear surface 108 towards the front surface 106 along the anterior or top end 102 thereof. In addition, the integrated femoral bone alignment device 100 may include an anatomical reference marker 122b extending from the anterior or top end 102 to the posterior or bottom end 104 along the front surface 106 thereof. During use, the anatomical reference markers 122 may be used to help determine internal-external (I-E) and medial-lateral (M-L) placement of the unicompartmental femoral implant. As will be appreciated by one of ordinary skill in the art, the anatomical reference marker 122 may take any suitable form now known or hereafter developed including, for example, a series of lines, grooves, slots, markings, or the like, or a combination thereof.

In addition, and/or alternatively, as shown, the integrated femoral bone alignment device 100 may also include a coupling mechanism 124 for coupling, engaging, etc. one or more shims or spacers, as will be shown and described in greater detail below. Thus arranged, a shim may be coupled to the integrated femoral bone alignment device 100 for positioning the integrated femoral bone alignment device 100 into the space between the patient's prepared tibial bone and unprepared femoral bone to help establish alignment of the unicompartmental femoral implant. The coupling mechanism 124 may be any now known or hereafter developed coupling mechanism for coupling a shim to the integrated femoral bone alignment device 100. As shown, in one embodiment, the coupling mechanism 124 may be in the form of a T-shaped member for slidably receiving the shim. Alternatively, the coupling mechanism could be in the form of a dovetail, a keyed arrangement, or the like.

In addition, and/or alternatively, as shown, the integrated femoral bone alignment device 100 may also include one or more additional drill guides 115 for guiding a drill to, for example, prepare the patient's bone to receive a fixation feature for the unicompartmental femoral implant. In addition, and/or alternatively, as shown, the integrated femoral bone alignment device 100 may also include one or more holes 117 for receiving a fixation member for provisionally coupling the integrated femoral bone alignment device 100 to the patient's bone. For example, in one embodiment, the hole 117 may be arranged and configured to receive a pin for provisionally coupling the integrated femoral bone alignment device 100 to the patient's bone, although it is envisioned that other fixation members may be used.

Exemplary methods of preparing a patient's femur will now be described. In various embodiments, the methods may be performed using, for example, the integrated femoral bone alignment device 100 described above in connection with FIG. 1. However, it will be appreciated, that the methods may be performed using different bone alignment devices. As such, the exemplary methods should not be limited to use with the integrated femoral bone alignment device 100 described above.

As will be described herein, in use, the exemplary methods utilize an integrated femoral bone alignment device that incorporates an alignment guide (e.g., drill guide) and a posterior cutting slot such as, for example, the integrated femoral bone alignment device 100. In addition, the exemplary methods may utilize associated instruments to evaluate the joint space between a patient's unprepared femoral bone and the patient's prepared tibial bone. Optional instruments may also be used to establish reference between an intramedullary alignment rod and the integrated femoral bone alignment device, and instruments to drill holes using the integrated femoral bone alignment device. In addition, instruments may be used to utilize the references created by the femoral bone alignment device, to prepare the distal surface of the femoral bone as an establishment of extension space balance.

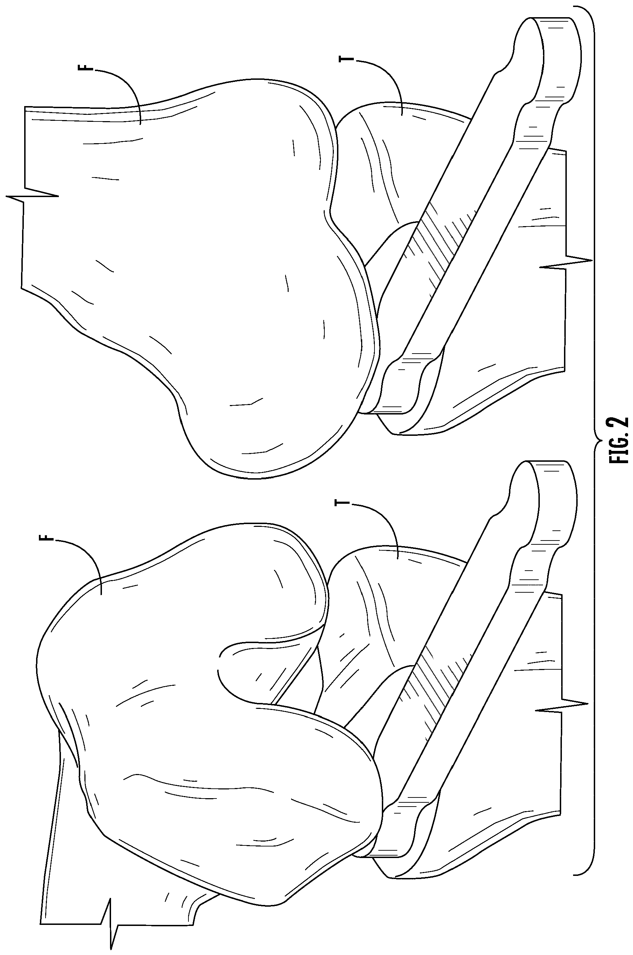

Referring to **FIG. 2**, reference will be made to a patient's knee undergoing partial knee reconstruction. In the illustrated examples, a 2mm imbalance exists between the unprepared femur or femoral bone F and the prepared tibia or tibial bone T. That is, a 2mm imbalance exists whereby the unprepared femur F influences the joint to have an additional 2mm of laxity in extension that is not present in flexion. Figures presented in connection with the example methods disclosed herein are shown with a configuration to correct this imbalance, however, as will be appreciated by one of ordinary skill in the art, this is but one example, and a patient's knee may include more or less imbalance between the unprepared femur F and the prepared tibia T.

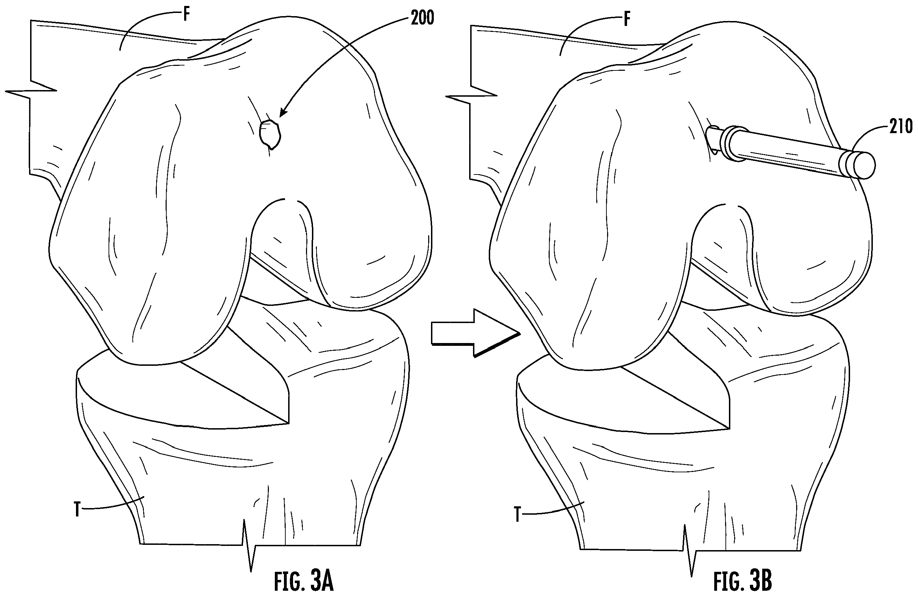

Referring to **FIGS. 3A-3G**, in connection with a first embodiment of an exemplary method for preparing a patient's femur F utilizing an integrated femoral bone alignment device such as, integrated femoral bone alignment device 100, will now be described.

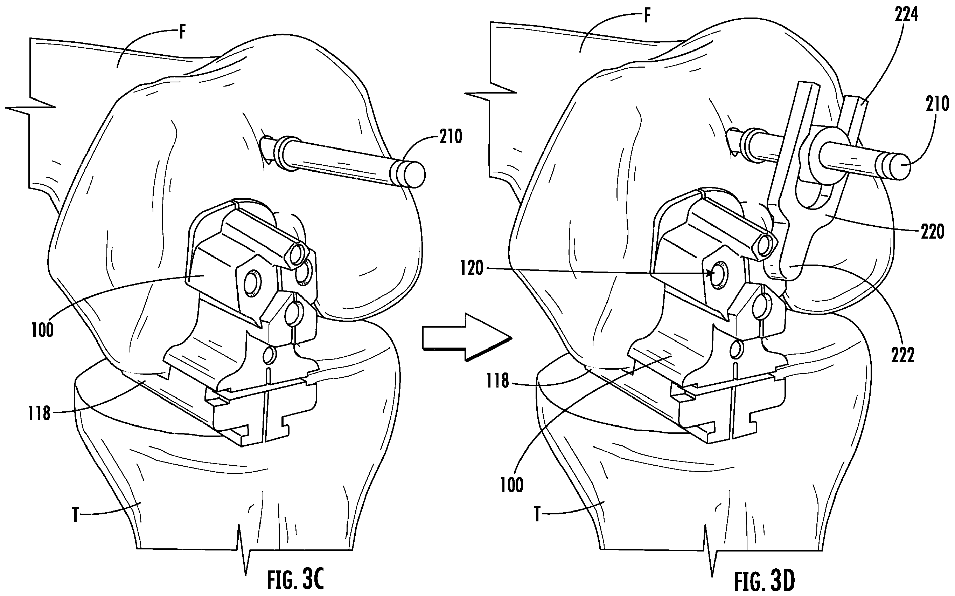

Referring to **FIG. 3A**, an access hole 200 is drilled into the distal end of the patient's femur F and into the intramedullary canal located within the femur F. Thereafter, as shown in **FIG. 3B**, an intramedullary alignment rod 210 is positioned into the access hole 200. Next, as shown in **FIG. 3C**, an integrated femoral bone alignment device such as, for example, integrated femoral bone alignment device 100, may be provisionally positioned by, for example, a surgeon's hand, against the patient's unprepared femoral bone F. For example, in one embodiment, a tongue 118 extending from the rear surface 108 of the integrated femoral bone alignment device 100 may be positioned in the space between the patient's unprepared femur F and the patient's tibia T for provisionally positioning the integrated femoral bone alignment device 100 against the patient's bones.

Referring to **FIG. 3D**, an alignment device 220 may be positioned and coupled to the integrated femoral bone alignment device 100 and the intramedullary rod 210. During use, the alignment device 220 may be used to establish alignment with respect to the femur concerning flexion-extension (F-E) and varus-valgus (V/V) alignment of the component (e.g., unicompartmental femoral implant). Meanwhile, anterior-posterior (A-P) placement may be established by contact with the tongue 118 extending from the rear surface 108 of the integrated femoral bone alignment device 100. Superior-inferior (S-I) placement may be established by contact with the rear surface 108 of the integrated femoral bone alignment device 100. The remaining two degrees of freedom with respect to internal-external (I-E), and medial-lateral (M-L) placement may be established by hand, potentially with aid of one or more anatomical reference markers 122a, 122b on the integrated femoral bone alignment device 100. In addition, as will be described in greater detail below, one or more spacers or shims 300 may be positioned between the integrated femoral bone alignment device 100 and the patient's prepared tibial bone T. In one embodiment, the spacer or shim 300 may be used to determine the distance between the unprepared femoral bone F and the prepared tibial bone T. During use, the spacer or shim 300 may be used to assist with establishing alignment with respect to the patient's femur concerning I-E rotation, and providing resistance to aid in establishment of the remaining degrees of freedom.

As shown, in one embodiment, the alignment device 220 may include a first end 222 and a second end 224, the first end 222 may be arranged and configured to engage the integrated femoral bone alignment device 100 while the second end 224 may be arranged and configured to engage the intramedullary alignment rod 210. While the alignment device 220 may be coupled to the integrated femoral bone alignment device 100 and the intramedullary alignment rod 210 by any now known or hereafter developed mechanisms, in one embodiment, as shown, the first end 222 may include a projection extending therefrom for receipt within the one or more holes or openings 120 formed in the integrated femoral bone alignment device 100. Meanwhile, the second end 224 may be arranged in a fork-like configuration having a space for receiving the intramedullary alignment rod 210. A fastener may subsequently be used to coupe the rod 210 to the alignment guide 220.

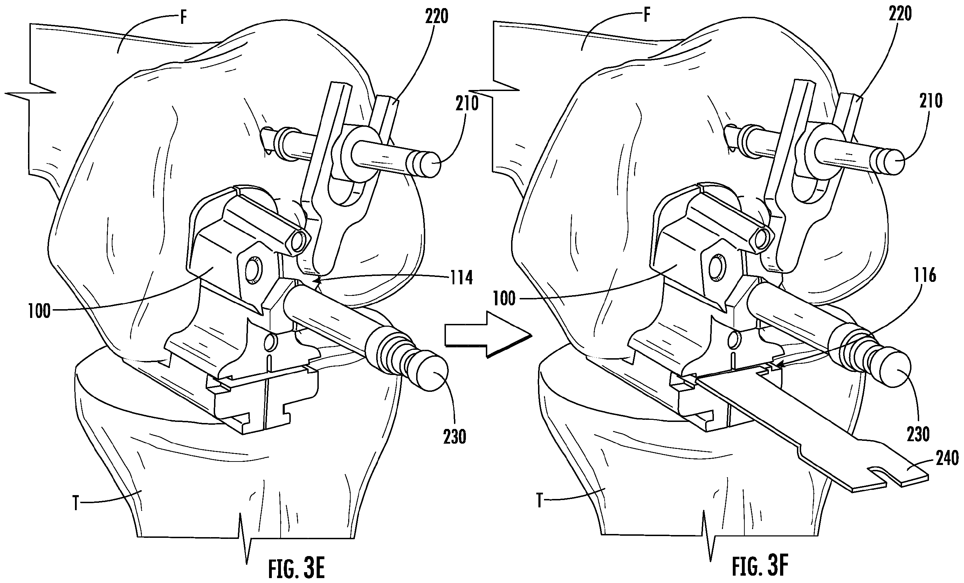

Referring to **FIG. 3E**, using the drill guide 114 formed in the integrated femoral bone alignment device 100, a drill bit 230 may be used to drill, form, create, etc. an alignment hole 235 in the distal portion of the patient's femur F. During use, the alignment hole 235 may be used by a preparation device to establish extension space balance (e.g., alignment hole 235 may be subsequently used as a guide for a separate cutting device as will be described in greater detail below). Referring to **FIG. 3F**, using the cutting slot 116 formed in the integrated femoral bone alignment device 100, a saw or cutting blade 240 may be used to resection the patient's femur F to establish flexion space balance by removing a known amount (thickness) of a patient's bone measured relative to a known implant thickness. For example, if the implant thickness is 6mm, the distance from the tongue 118 to the cutting slot 116 would be 6mm to replace the bone removed, or 8mm (or some other amount) if additional (or less) laxity was desired. In any event, the distance from the tongue 118 to the cutting slot 116 would be a fixed distance set into the integrated femoral bone alignment device 100 (e.g., the integrated femoral bone alignment device 100 would always resect implant thickness, or 2mm more, etc. as designed).

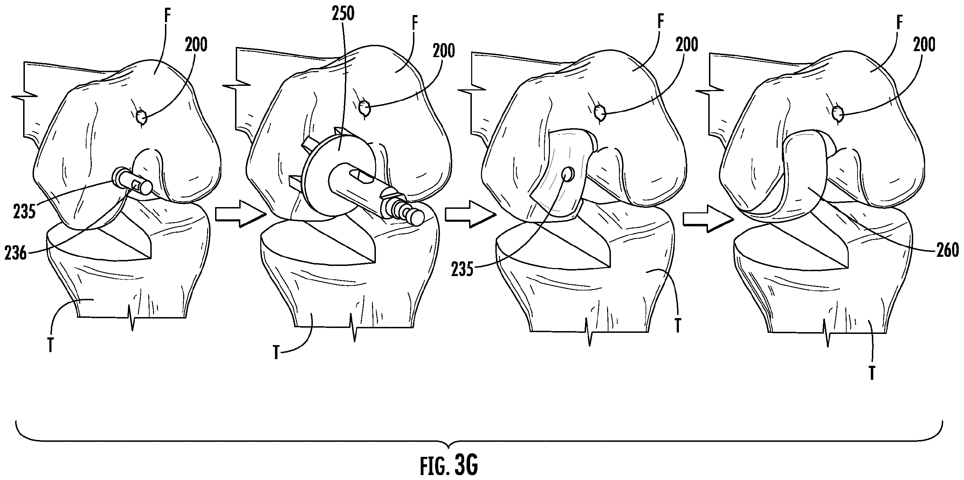

Once these steps are completed, referring to FIG. 3G, the integrated femoral bone alignment device 100 may be removed and the distal portion of the patient's femur F may be prepared using the established reference points such as, for example, the alignment hole 235 may be used to position an alignment shaft 236 for receiving a rotary mill or cutting tool 250. In addition, femoral trialing may be completed, and a trial implant affixed 260 or an orthopedic implant may be implanted.

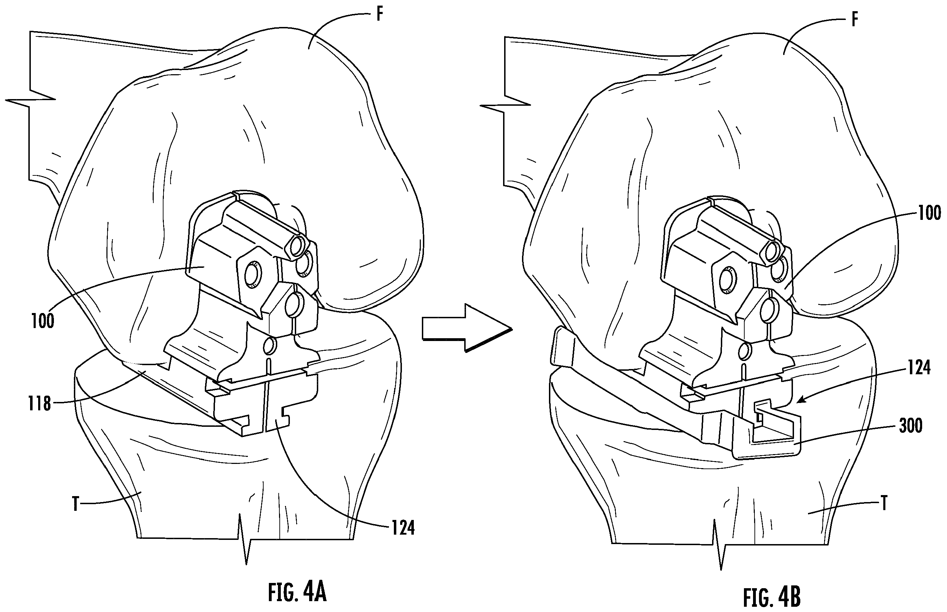

Referring to **FIGS. 4A-4E**, a second embodiment of a method for preparing a patient's femur utilizing an integrated femoral bone alignment device such as, integrated femoral bone alignment device 100, will now be described. As will be shown and described, the second exemplary method is substantially similar to the first exemplary method. As will be shown and described, in use, the second exemplary method may be performed without drilling the access hole 200 into the distal end of the patient's femur F for receiving the intramedullary alignment rod 210 for coupling the alignment device 220 to the integrated femoral bone alignment device 100.

In connection with the second embodiment, referring to **FIG. 4A**, the integrated femoral bone alignment device 100 may be initially provisionally positioned against the patient's unprepared femoral bone F. For example, the tongue 118 extending from the rear surface 108 of the integrated femoral bone alignment device 100 may be positioned in the space between the patient's unprepared femur F and the patient's tibia T for provisionally positioning the integrated femoral bone alignment device 100 against the patient's bones.

Referring to **FIG. 4B**, one or more spacers or shims 300 (used interchangeably herein without the intent to limit) may be positioned between the integrated femoral bone alignment device 100 and the patient's prepared tibia T. In one embodiment, the shim 300 may be used to determine the distance between the unprepared femoral bone F and the prepared tibial bone T. During use, the shim 300 may be used to assist with establishing alignment with respect to the patient's femur concerning I-E rotation, and providing resistance to aid in establishment of the remaining degrees of freedom. For example, in use, the shims 300 fill the space between the integrated femoral bone alignment device 100 and the patient's prepared tibia bone T creating residual tension provided by the soft tissue structures to stabilize the relative position of the bones to each other, and the alignment device to the femoral bone by holding its location to the tibia T. F-E, and V/V alignment of the component (e.g., unicompartmental femoral implant) are determined by the relative positioning between the patient's femur F and tibia T bones. In one embodiment, the shim 300 may be positioned into engagement with the coupling mechanism 124 formed on the integrated femoral bone alignment device 100 and slide into the space between the patient's prepared tibial bone T and the patient's unprepared femoral bone F.

As previously described in connection with the method of **FIGS. 3A-3G**, A-P placement may be established by contact with the tongue 118 extending from the rear surface 108 of the integrated femoral bone alignment device 100, and S-I placement may be established by contact with the rear surface 108 of the integrated femoral bone alignment device 100. The remaining degree of freedoms, with respect to M-L, F/E, and V/V placement may be established by hand, potentially with aid of the anatomical reference markers 122a, 122b formed on the integrated femoral bone alignment device 100. In use, the anatomical reference markers 122a, 122b may be aligned with the center of the prepared tibia T.

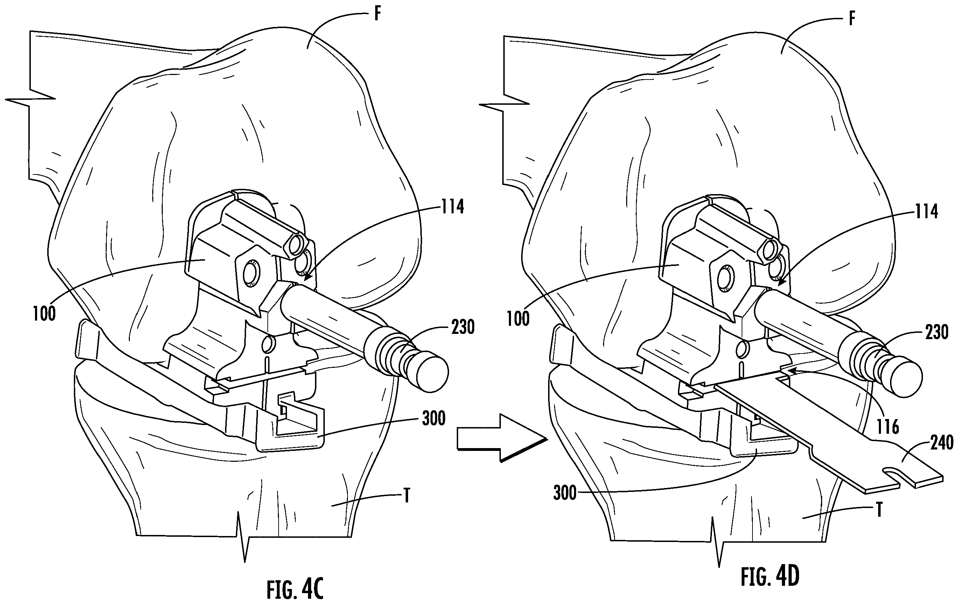

Next, referring to **FIG. 4C**, similar to steps previously described, using the drill guide 114 formed in the integrated femoral bone alignment device 100, a drill bit 230 may be used to drill, form, create, etc. an alignment hole 235 in the distal portion of the patient's femur F. During use, the alignment hole 235 may be used by a preparation device to establish extension space balance (e.g., alignment hole 235 may be subsequently used as a guide for a separate cutting device). Referring to **FIG. 4D**, using the cutting slot 116 formed in the integrated femoral bone alignment device 100, a saw or cutting blade 240 may be used to resection the patient's femur F to establish flexion space balance by removing a known amount (thickness) of a patient's bone measured relative to a known implant thickness. For example, as previously described, if the implant thickness is 6mm, the distance from the tongue 118 to the cutting slot 116 would be 6mm to replace the bone removed, or 8mm (or some other amount) if additional (or less) laxity was desired. In any event, the distance from the tongue 118 to the cutting slot 116 would be a fixed distance set into the integrated femoral bone alignment device 100 (e.g., the integrated femoral bone alignment device 100 would always resect implant thickness, or 2mm more, etc. as designed).

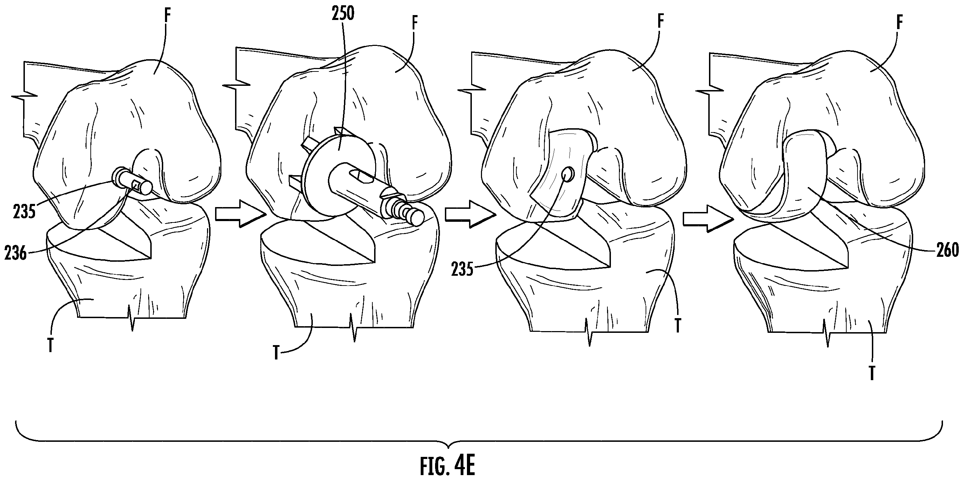

Once these steps are completed, referring to **FIG. 4E**, the integrated femoral bone alignment device 100 may be removed and the distal portion of the patient's femur F may be prepared using the established reference points such as, for example, the alignment hole 235 may be used to position an alignment shaft 236 for receiving a rotary mill or cutting tool 250. In addition, femoral trialing may be completed, and a trial implant affixed 260 or an orthopedic implant may be implanted.

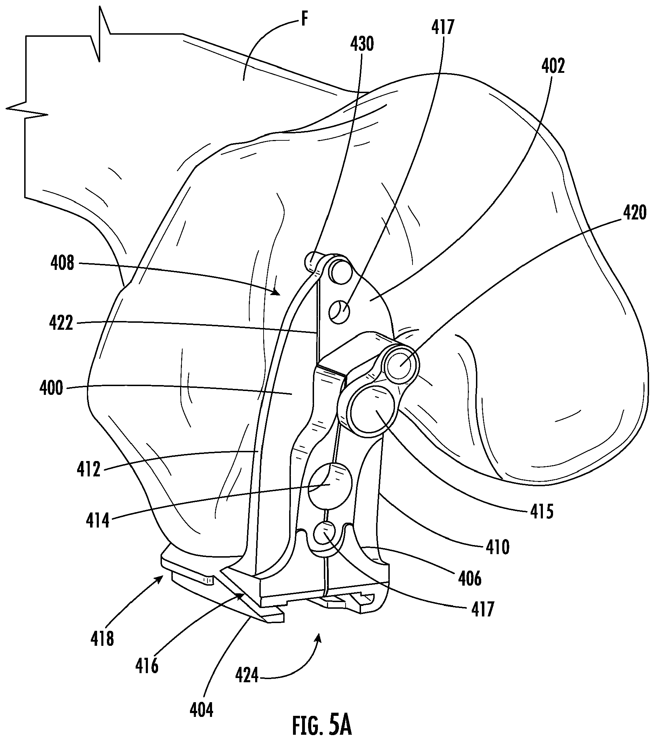

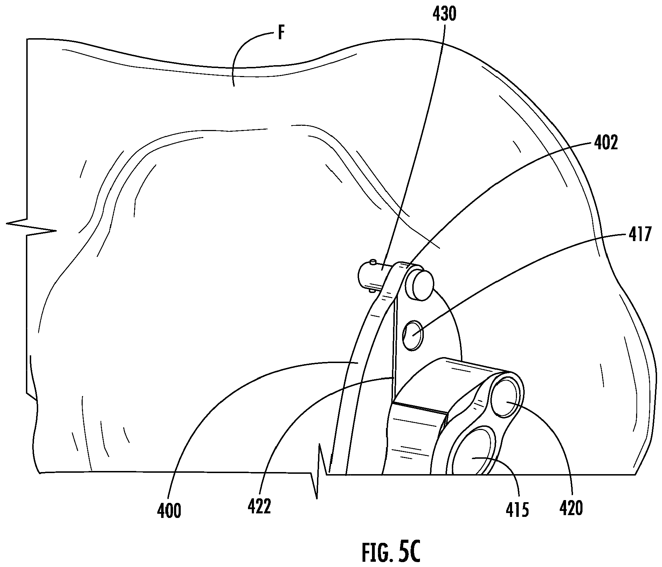

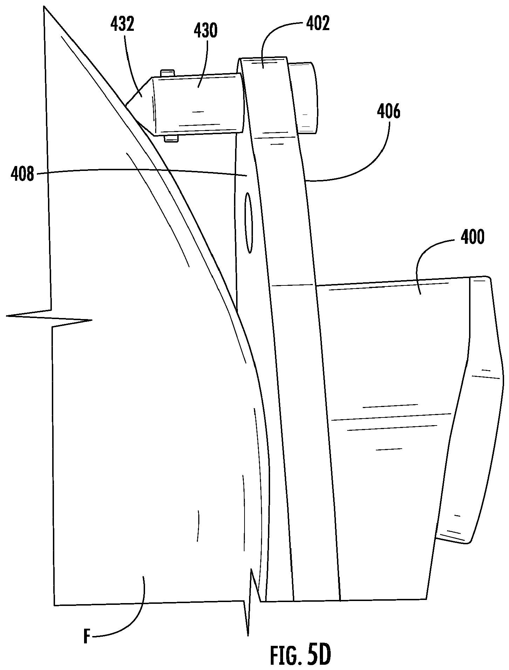

**FIGS. 5A-5D** illustrate an alternate embodiment of a bone alignment device such as, for example, an integrated femoral bone alignment device 400 for use in preparing a patient's femoral bone undergoing a partial knee reconstruction procedure. Similar to the embodiment previously described, the integrated femoral bone alignment device 400 may integrate a femoral alignment guide (e.g., a drill guide), a cutting or sawing slot, and references to establish alignment to the unprepared distal and posterior femoral bone, and may be used in connection with the methods previously described. The integrated femoral bone alignment device 400 however may be characterized as a handed device. That is, the integrated femoral bone alignment device 400 is arranged and configured to contact either the medial or lateral portion of the patient's femoral bone (e.g., the outer profile of the integrated femoral bone alignment device 400 is asymmetric and arranged and configured for use with either the medial or lateral portion of the patient's femur). For example, as shown, the integrated femoral bone alignment device 400 is arranged and configured to contact a lateral portion of the patient's bone (e.g., femur). A similar device may also be provided for contacting a medial portion of the patient's bone (e.g., femur).

As shown, the integrated femoral bone alignment device 400 includes an anterior or top end 402, a posterior or bottom end 404, a front surface 406, a rear surface 408, a medial or first side surface 410, and a lateral or second side surface 412. In addition, as shown, the integrated femoral bone alignment device 400 may also include one or more alignment or drill guides 414 for establishing alignment of a unicompartmental femoral implant as previously described. As shown, the one or more drill guides 414 extend from the rear surface 408 to the front surface 406 and may be used to prepare, for example, a primary peg for subsequent preparation steps).

In addition, and/or alternatively, as shown, the integrated femoral bone alignment device 400 may also include one or more cutting slots 416 for preparing a portion of the patient's femoral bone, as previously described. As shown, the cutting slot 416 may extend from the rear surface 408 to the front surface 406, and from the first side surface 410 to the second side surface 412.

In addition, and/or alternatively, as shown, the integrated femoral bone alignment device 400 may also include a tongue, projection or foot 418 (used interchangeably herein without the intent to limit) extending from the rear surface 408 of the device 400. As will be described in greater detail below, during use, the tongue 418 is arranged and configured to be positioned in the space between the patient's unprepared femoral bone and the patient's tibial bone for provisionally positioning the integrated femoral bone alignment device 400 against the patient's bones.

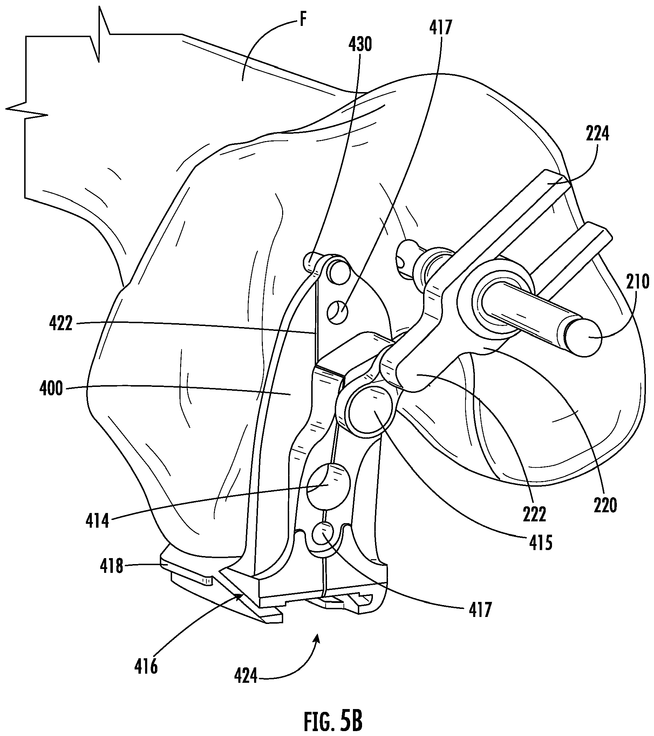

In addition, and/or alternatively, the integrated femoral bone alignment device 400 may include one or more holes or openings 420 for connecting the integrated femoral bone alignment device 400 to an optional intramedullary alignment rod 210 (as illustrated in **FIG. 5B**). As shown, the one or more holes or openings 420 may extend from the front surface 406 towards the rear surface 408. As shown, the opening 420 may be positioned adjacent to the medial or first side surface 410 for the lateral, integrated femoral bone alignment device 400. Alternatively, the opening 420 may be positioned adjacent to the lateral or second side surface 412 for the medial, integrated femoral bone alignment device 400.

In addition, and/or alternatively, the integrated femoral bone alignment device 400 may also include one or more anatomical reference markers 422, as previously described, to assist the surgeon in determining one or more degrees of freedom. For example, as previously described, the integrated femoral bone alignment device 400 may include an anatomical reference marker 422 extending from the anterior or top end 402 to the posterior or bottom end 404 along the front surface 406 thereof. In addition, the integrated femoral bone alignment device 400 may include an anatomical reference marker (not shown) extending from the rear surface 408 towards the front surface 406 along the anterior or top end 402 thereof. During use, the anatomical reference markers 422 may be used to help determine internal-external (I-E) and medial-lateral (M-L) placement of the unicompartmental femoral implant. As will be appreciated by one of ordinary skill in the art, the anatomical reference marker 422 may take any suitable form now known or hereafter developed including, for example, a series of lines, grooves, slots, markings, or the like, or a combination thereof.

In addition, and/or alternatively, as shown, the integrated femoral bone alignment device 100 may also include a coupling mechanism 424 for coupling, engaging, etc. one or more shims or spacers, as previously described. Thus arranged, a shim may be coupled to the integrated femoral bone alignment device 400 for positioning the integrated femoral bone alignment device 400 into the space between the patient's prepared tibia and unprepared femur to help establish alignment of the unicompartmental femoral implant. The coupling mechanism 424 may be any now known or hereafter developed coupling mechanism for coupling a shim to the integrated femoral bone alignment device 400. As shown, in one embodiment, the coupling mechanism 424 may be in the form of a slot or female connection to slidably receive the shim, spacer, or the like. Alternatively, the coupling mechanism could be in the form of a male connection member such as, for example, a T-shaped member, a dovetail, a keyed arrangement, or the like.

In addition, and/or alternatively, as shown, the integrated femoral bone alignment device 400 may also include one or more additional drill guides 415 for guiding a drill to, for example, prepare the patient's bone to receive a fixation feature for the implant (e.g., opening to prepare, for example, a secondary peg). In addition, and/or alternatively, as shown, the integrated femoral bone alignment device 400 may also include one or more holes 417 for receiving a fixation member for provisionally coupling the integrated femoral bone alignment device 400 to the patient's bone. For example, in one embodiment, the hole 417 may be arranged and configured to receive a pin for provisionally coupling the integrated femoral bone alignment device 400 to the patient's bone, although it is envisioned that other fixation members may be used.

In addition, as shown, the integrated femoral bone alignment device 100 may include an indicator 430 having a tip 432 for contacting the patient's bone. In use, the configuration (e.g., angle, size, or the like) of the integrated femoral bone alignment device 400 may be selected to ensure that the tip 432 of the device 400 doesn't sit proud (or too deep) of the anatomy after bone preparation is completed.

As described herein, the example methods pre-balance the patient's knee to eliminate sequential preparation steps of the distal portion of the patient's femur, however it is envisioned that the methods may be used without pre-balancing the knee and may establish knee balancing via traditional sequential preparation.

In addition, as illustrated herein, the example methods are illustrated and described in connection with devices having a single articular radius, singular bone facing radius, and a single positive fixation feature, however it is envisioned that the methods could be used to prepare a patient's bone for receiving a device with multiple bone facing or articulating radii, and with multiple, or no positive fixation features.

While the femoral bone alignment devices have been shown and described as including a number of different features, it is envisioned that the femoral bone alignment devices need not include each and every feature described. For example, it is envisioned that femoral bone alignment devices may only include a subset of the features described. For example, the femoral bone alignment devices need not include one or more holes or openings for coupling to an alignment device, one or more anatomic markers, a coupling mechanism for coupling to a shim, etc.

In addition, it is envisioned that the methods described herein may be modified to hold extension space balance constant, to allow variation in flexion space balance, or hold neither constant and allow for varying both extension and flexion balance through modification to femur preparation. Additionally, or alternatively, in some embodiments, multiple integrated femoral bone alignment devices with different resection levels (e.g. 6mm, 8mm, 10mm) could be provided, for example, in a kit. Alternatively, an integrated femoral bone alignment device with multiple cutting slots could be provided. In another embodiment, spacers or shims that would have material anterior to tongue could be utilized.

In accordance with principles of the present invention, the femoral bone alignment devices allow femoral component alignment with an integrated drilling and cutting guide in a single, unitary body. That is, in accordance with the principles described herein, a novel instrument and accompanying methods to prepare a patient's femur to receive an orthopedic implant with a curved distal surface in fewer surgical steps has been disclosed.

In accordance with the present disclosure, pre-balancing the patient's knee such that flexion and extension space balance, bone preparation, and sizing can be completed with a single integrated device. Thus arranged, an integrated bone alignment/sizing device for the femur is provided. With the aid of a set of mechanically coupled tensioning devices, the integrated femoral bone alignment device can set the location of the femoral component (e.g., unicompartmental femoral implant) relative to joint tension with reference to a prepared tibia surface. Sizing of the femoral component can also be determined/confirmed by provided references integrated into the femoral bone alignment device. Posterior resection/joint space establishment can also be completed using the integrated femoral bone alignment device. Thus arranged, the integrated femoral bone alignment device can achieve femoral alignment with respect to anatomic landmarks, with or without reference to an intramedullary alignment device.

In addition, the femoral bone alignment device enables a plurality of shims or spacers to be mechanically coupled to the device to provide alignment with reference to a patient's prepared tibia.

In addition, the femoral alignment device enables alignment with or without an intramedullary rod reference to aid in the alignment component anterior shape is provided in its entirety, along with a marking to reference center of articulation with consideration of fixation features.

Thus arranged, the integrated femoral bone alignment device facilitates corresponding methods of preparing a patient's femur requiring fewer steps to prepare the femur to receive a trial / implant. For example, steps may be reduced in one or more of the following ways: pre-balancing the knee to eliminate additional steps associated with sequential reaming, milling, or range of motion reaming (e.g., extension space balance can be established by a single reaming/milling step); combination of providing a cutting slot into the guide, eliminates the need for additional guides to complete posterior bone preparation; providing references in a single guide with respect to anterior component shape, center of articulation (and fixation features), and to receive a spacer for alignment without reference to an intramedullary guide, enables optional elimination of the need to violate the intramedullary canal, and the steps associated with creating the intramedullary reference.

While the present disclosure refers to certain embodiments, numerous modifications, alterations, and changes to the described embodiments are possible without departing from the sphere and scope of the present disclosure, as defined in the appended claim(s). Accordingly, it is intended that the present disclosure not be limited to the described embodiments, but that it has the full scope defined by the language of the following claims, and equivalents thereof. The discussion of any embodiment is meant only to be explanatory and is not intended to suggest that the scope of the disclosure, including the claims, is limited to these embodiments. In other words, while illustrative embodiments of the disclosure have been described in detail herein, it is to be understood that the inventive concepts may be otherwise variously embodied and employed, and that the appended claims are intended to be construed to include such variations, except as limited by the prior art.

The foregoing discussion has been presented for purposes of illustration and description and is not intended to limit the disclosure to the form or forms disclosed herein. For example, various features of the disclosure are grouped together in one or more embodiments or configurations for the purpose of streamlining the disclosure. However, it should be understood that various features of the certain embodiments or configurations of the disclosure may be combined in alternate embodiments, or configurations. Moreover, the following claims are hereby incorporated into this Detailed Description by this reference, with each claim standing on its own as a separate embodiment of the present disclosure.

The device and associated methods in accordance with the present disclosure have been described with reference to particular embodiments thereof. Therefore, the above description is by way of illustration and not by way of limitation. Accordingly, it is intended that all such alterations and variations and modifications of the embodiments are within the scope of the present invention as defined by the appended claims.

While the methods disclosed herein have been described and shown with reference to particular steps performed in a particular order, it will be understood that these steps may be combined, subdivided, or re-ordered to form an equivalent method without departing from the teachings of the present disclosure. Accordingly, unless specifically indicated herein, the order and grouping of the steps are not generally intended to be a limitation of the present disclosure.

As used herein, an element or step recited in the singular and proceeded with the word "a" or "an" should be understood as not excluding plural elements or steps, unless such exclusion is explicitly recited. Furthermore, references to "one embodiment" of the present disclosure are not intended to be interpreted as excluding the existence of additional embodiments that also incorporate the recited features.

The phrases "at least one", "one or more", and "and/or", as used herein, are openended expressions that are both conjunctive and disjunctive in operation. The terms "a" (or "an"), "one or more" and "at least one" can be used interchangeably herein. All directional references (e.g., proximal, distal, upper, lower, upward, downward, left, right, lateral, longitudinal, front, back, top, bottom, above, below, vertical, horizontal, radial, axial, clockwise, and counterclockwise) are only used for identification purposes to aid the reader's understanding of the present disclosure, and do not create limitations, particularly as to the position, orientation, or use of this disclosure. Connection references (e.g., engaged, attached, coupled, connected, and joined) are to be construed broadly and may include intermediate members between a collection of elements and relative to movement between elements unless otherwise indicated. As such, connection references do not necessarily infer that two elements are directly connected and in fixed relation to each other. All rotational references describe relative movement between the various elements. Identification references (e.g., primary, secondary, first, second, third, fourth, etc.) are not intended to connote importance or priority but are used to distinguish one feature from another. The drawings are for purposes of illustration only and the dimensions, positions, order and relative to sizes reflected in the drawings attached hereto may vary.

### CLAUSES

1. An integrated femoral bone alignment device arranged and configured to prepare a patient's unprepared femoral bone in a partial knee reconstruction surgery to receive a unicompartmental femoral implant, the device comprising:
   a top end, a bottom end, a front surface, a rear surface, a medial side surface, and a lateral side surface;
   a femoral alignment drill guide extending from the rear surface to the front surface, the femoral alignment drill guide arranged and configured to form an alignment hole in a distal end of the patient's femoral bone to establish alignment of the unicompartmental femoral implant;
   a cutting slot extending from the rear surface to the front surface, the cutting slot being arranged and configured to receive a surgical cutting instrument for resecting a portion of the patient's unprepared femoral bone; and
   one or more anatomical reference markers to establish alignment of the integrated femoral bone alignment device, and hence the unicompartmental femoral implant, relative to patient's unprepared femoral bone.
2. The integrated femoral bone alignment device of clause 1, wherein the one or more anatomical reference markers includes:
   a first anatomical reference marker extending along a top end of the device from the rear surface towards the front surface; and
   a second anatomical reference marker extending along a front surface of the device from the top end to the bottom end.
3. The integrated femoral bone alignment device of clause 2, wherein, during use, the first and second anatomical reference markers assist a surgeon determine internal-external (I-E) and medial-lateral (M-L) placement of the unicompartmental femoral implant
4. The integrated femoral bone alignment device of clause 1, wherein the anatomical reference markers consists one of a series of lines, grooves, slots, markings, or a combination thereof.
5. The integrated femoral bone alignment device of clause 1, further comprising a tongue extending from the rear surface, the tongue being arranged and configured to be positioned in a space between the patient's unprepared femoral bone and a patient's prepared tibial bone for provisionally positioning the integrated femoral bone alignment device against the patient's femoral bone.
6. The integrated femoral bone alignment device of clause 5, wherein a distance between the tongue and the cutting slot is a fixed distance, such that resecting a portion of the patient's unprepared femoral bone removes a known thickness of the patient's femoral bone.
7. The integrated femoral bone alignment device of clause 5, wherein positioning the tongue in the space between the patient's unprepared femoral bone and the patient's tibial bone establishes anterior-posterior (A-P) placement of the unicompartmental femoral implant.
8. The integrated femoral bone alignment device of clause 1, further comprising:
   one or more openings formed in the front surface thereof; and
   an alignment guide arranged and configured to be positioned within the one or more openings for coupling the integrated femoral bone alignment device to an alignment rod referencing the patient's femoral bone.
9. The integrated femoral bone alignment device of clause 7, wherein the integrated femoral bone alignment device includes first and second openings positioned on either side of the integrated femoral bone alignment device.
10. The integrated femoral bone alignment device of clause 7, wherein, in use, coupling the alignment guide with the integrated femoral bone alignment device and the alignment rod establishes flexion-extension (F-E) and varus-valgus (V/V) alignment of the unicompartmental femoral implant.
11. The integrated femoral bone alignment device of clause 1, further comprising a coupling mechanism for one or more shims, the one or more shims being arranged and configured to slidably engage with the coupling mechanism for positioning the integrated femoral bone alignment device into a space between a patient's prepared tibial bone and the patient's unprepared femoral bone establishes alignment of the unicompartmental femoral implant.
12. The integrated femoral bone alignment device of clause 9, wherein the coupling mechanism is in the form of a T-shaped member for slidably receiving the one or more shims.
13. The integrated femoral bone alignment device of clause 9, wherein the coupling mechanism is in the form of a slot or female connection for slidably receiving the one or more shims.
14. The integrated femoral bone alignment device of clause 1, further comprising an indicator having a tip for contacting the patient's bone.
15. The integrated femoral bone alignment device of clause 1, further comprising one or more additional drill guides arranged and configured to receive a surgical drill to prepare the patient's bone to receive a fixation feature for the unicompartmental femoral implant.
16. The integrated femoral bone alignment device of clause 1, further comprising one or more holes arranged and configured to receive a fixation member for provisionally coupling the integrated femoral bone alignment device to the patient's unprepared femoral bone.
17. The integrated femoral bone alignment device of clause 14, wherein the fixation member is a pin for provisionally coupling the integrated femoral bone alignment device to the patient's unprepared femoral bone.
18. The integrated femoral bone alignment device of clause 1, wherein an outer profile of the integrated femoral bone alignment device is asymmetric and arranged and configured for use with either a medial or a lateral portion of the patient's femoral bone.
19. The integrated femoral bone alignment device of clause 1, wherein superior-inferior (S-I) placement of the unicompartmental femoral implant is established by contacting the rear surface of the integrated femoral bone alignment device with the patient's unprepared femoral bone.
20. A method for preparing a patient's femoral bone for use in a partial knee reconstruction surgery to implant a unicompartmental femoral implant, the patient's knee having an existing imbalance in the patient's unprepared femoral bone in extension as compared to flexion, the method comprising:
   positioning a tongue extending from a rear surface of an integrated femoral bone alignment device into a patient's knee space between the patient's unprepared femoral bone and a patient's prepared tibial bone, the integrated femoral bone alignment device further including an alignment guide and a cutting slot;
   inserting a drill into the alignment guide to form an alignment hole in a distal end of the patient's unprepared femoral bone;
   inserting a saw blade into the cutting slot to resect a posterior portion of the patient's femoral bone;
   removing the integrated femoral bone alignment device; and
   preparing the patient's femur utilizing the alignment hole.
21. The method of clause 20, further comprising:
   forming an access hole into a distal end of the patient's unprepared femoral bone;
   inserting an alignment rod into the access hole; and
   coupling an alignment device to the integrated femoral bone alignment device and to the alignment rod.
22. The method of clause 21, wherein coupling the alignment device to the integrated femoral bone alignment device and to the alignment rod comprises:
   inserting a projection formed on a first end of the alignment device into a hole formed in the integrated femoral bone alignment device; and
   positioning a fork-like second end portion of the alignment device about the alignment rod.
23. The method of clause 21, wherein coupling the alignment device to the integrated femoral bone alignment device and the alignment rod establishes flexion-extension (F-E) and varus-valgus (V/V) alignment of the unicompartmental femoral implant.
24. The method of clause 20, further comprising:
   inserting an alignment shaft into the alignment hole; and
   positioning a rotary cutting tool over the alignment shaft to prepare the patient's unprepared femoral bone.
25. The method of clause 20, further comprising inserting one or more shims within the patient's knee space between the integrated femoral bone alignment device and the patient's prepared tibial bone to establish alignment of the unicompartmental femoral implant with respect to the patient's femoral bone, the one or more shims being coupled to the integrated femoral bone alignment device.
26. The method of clause 20, wherein a distance between the tongue and the cutting slot is a fixed distance such that resecting a posterior portion of the patient's unprepared femoral bone removes a known thickness of the patient's femoral bone.
27. The method of clause 20, wherein the integrated femoral bone alignment device further includes a first anatomical reference marker extending along a top end of the integrated femoral bone alignment device and a second anatomical reference marker extending along a front surface of the integrated femoral bone alignment device, the method further comprises:
   using the first and second anatomical reference markers to establish internal-external (I-E) and medial-lateral (M-L) placement of the unicompartmental femoral implant.
28. The method of clause 20, wherein positioning the tongue into the patient's knee space establishes anterior-posterior (A-P) placement of the unicompartmental femoral implant.
29. The method of clause 20, wherein positioning the tongue into the patient's knee space comprises contacting a rear surface of the integrated femoral bone alignment device with the patient's unprepared femoral bone to establish superior-inferior (S-I) placement of the unicompartmental femoral implant.

## Claims

1. An integrated femoral bone alignment device arranged and configured to prepare a patient's unprepared femoral bone in a partial knee reconstruction surgery to receive a unicompartmental femoral implant, the device comprising:
a top end, a bottom end, a front surface, a rear surface, a medial side surface, and a lateral side surface;
a femoral alignment drill guide extending from the rear surface to the front surface, the femoral alignment drill guide arranged and configured to form an alignment hole in a distal end of the patient's femoral bone to establish alignment of the unicompartmental femoral implant; and
one or more anatomical reference markers to establish alignment of the integrated femoral bone alignment device, and hence the unicompartmental femoral implant, relative to patient's unprepared femoral bone;
first and second openings positioned on either side of the integrated femoral bone alignment device formed in the front surface thereof; and
an alignment guide arranged and configured to be positioned within the first or second openings for coupling the integrated femoral bone alignment device to an alignment rod referencing the patient's femoral bone.

2. The integrated femoral bone alignment device of claim 1, wherein the one or more anatomical reference markers includes:
a first anatomical reference marker extending along a top end of the device from the rear surface towards the front surface; and
a second anatomical reference marker extending along a front surface of the device from the top end to the bottom end.

3. The integrated femoral bone alignment device of claim 2, wherein, during use, the first and second anatomical reference markers assist a surgeon determine internal-external (I-E) and medial-lateral (M-L) placement of the unicompartmental femoral implant

4. The integrated femoral bone alignment device of claim 1, further comprising a tongue extending from the rear surface, the tongue being arranged and configured to be positioned in a space between the patient's unprepared femoral bone and a patient's prepared tibial bone for provisionally positioning the integrated femoral bone alignment device against the patient's femoral bone.

5. The integrated femoral bone alignment device of claim 4, wherein a distance between the tongue and the cutting slot is a fixed distance, such that resecting a portion of the patient's unprepared femoral bone removes a known thickness of the patient's femoral bone.

6. The integrated femoral bone alignment device of claim 4, wherein positioning the tongue in the space between the patient's unprepared femoral bone and the patient's tibial bone establishes anterior-posterior (A-P) placement of the unicompartmental femoral implant.

7. The integrated femoral bone alignment device of claim 1, wherein, in use, coupling the alignment guide with the integrated femoral bone alignment device and the alignment rod establishes flexion-extension (F-E) and varus-valgus (V/V) alignment of the unicompartmental femoral implant.

8. The integrated femoral bone alignment device of claim 1, further comprising a coupling mechanism for one or more shims, the one or more shims being arranged and configured to slidably engage with the coupling mechanism for positioning the integrated femoral bone alignment device into a space between a patient's prepared tibial bone and the patient's unprepared femoral bone establishes alignment of the unicompartmental femoral implant.

9. The integrated femoral bone alignment device of claim 8, wherein the coupling mechanism is in the form of a T-shaped member for slidably receiving the one or more shims.

10. The integrated femoral bone alignment device of claim 8, wherein the coupling mechanism is in the form of a slot or female connection for slidably receiving the one or more shims.

11. The integrated femoral bone alignment device of claim 1, further comprising one or more additional drill guides arranged and configured to receive a surgical drill to prepare the patient's bone to receive a fixation feature for the unicompartmental femoral implant.

12. The integrated femoral bone alignment device of claim 1, further comprising one or more holes arranged and configured to receive a fixation member for provisionally coupling the integrated femoral bone alignment device to the patient's unprepared femoral bone.

13. The integrated femoral bone alignment device of claim 1, further comprising an indicator having a tip for contacting the patient's bone, wherein the fixation member is a pin for provisionally coupling the integrated femoral bone alignment device to the patient's unprepared femoral bone.

14. The integrated femoral bone alignment device of claim 1, wherein an outer profile of the integrated femoral bone alignment device is asymmetric and arranged and configured for use with either a medial or a lateral portion of the patient's femoral bone.

15. The integrated femoral bone alignment device of claim 1, wherein superior-inferior (S-I) placement of the unicompartmental femoral implant is established by contacting the rear surface of the integrated femoral bone alignment device with the patient's unprepared femoral bone.
